# FASCICULE DE BREVET EUROPEEN

(11) **EP 2 522 384 B1**
(45) Date de publication et mention de la délivrance du brevet: **05.07.2017**
(21) Numéro de dépôt: 12166858.6
(22) Date de dépôt: 04.05.2012
(51) Int. Cl.: A61M 16/10, A61B 5/097, G01N 33/00, G01N 33/497, A61B 5/08, A61M 16/00

(54) **Appareil de distribution de NO avec ligne de calibration intégrée et installation associée**
NO-Verteilungsgerät mit integierter Kalibrierlinie und entsprechende Anlage
Apparatus for dispensing NO with built-in calibration line and related facility

(30) Priorité: 10.05.2011 FR 1153984
(43) Date de publication de la demande: 14.11.2012
(73) Titulaire: Air Liquide Medical Systems, 92160 Antony (FR)
(72) Inventeur: Boulanger, Thierry, 37000 Tours (FR)
(74) Mandataire: Pittis, Olivier

(56) Documents cités:
- EP-A1- 0 904 729
- EP-A2- 0 872 254
- JP-A- 8 200 612
- US-A- 5 558 083
- US-A1- 2006 207 594

## Description

L'invention porte sur un appareil de distribution de gaz, en particulier adapté à et conçu pour être raccordé entre une source de NO gazeux et le circuit ventilatoire d'un ventilateur d'administration de gaz, lequel comprend une ligne de calibration de gaz intégrée permettant d'opérer une calibration automatique de l'appareil.

L'oxyde nitrique ou monoxyde d'azote (ci après «NO») est un gaz incolore qui, lorsqu'il est inhalé, dilate les vaisseaux sanguins des poumons et augmente l'oxygénation du sang en favorisant les échanges gazeux. Cette propriété est exploitée pour traiter l'hypertension artérielle pulmonaire.

A cette fin, le NO est ajouté aux gaz inspirés par le patient souffrant d'hypertension pulmonaire selon une posologie déterminée, fixée par le médecin, qui varie généralement entre 1 et 100 ppm en volume, notamment en fonction du patient considéré.

Le gaz inhalé par le patient contenant du NO est fourni par un dispositif de délivrance, tel qu'un ventilateur mécanique, comme décrit par exemple par US-A-5,558,083.

Un tel appareil, qui est alimenté par une ou des bouteilles contenant un mélange d'azote (N₂) et de NO à une concentration typiquement de l'ordre de 200 à 800 ppm en volume de NO, comprend généralement un module d'injection de NO directement placé dans la branche inspiratoire d'un circuit patient connecté à ses extrémités respectivement au ventilateur mécanique et à un patient. Le module d'injection inclut un capteur de débit qui mesure le débit délivré par le ventilateur mécanique et retourne cette mesure à l'appareil d'administration de NO pour que celui-ci en déduise un débit de NO à délivrer, en relation avec la posologie désirée.

Le débit ainsi déterminé est assuré au moyen d'une électrovanne proportionnelle associée à un capteur de débit interne à l'appareil et est administré au moyen d'une ligne d'injection située dans le dit module d'injection, en aval du système de mesure. Le mode d'administration du NO est préférentiellement continu en ce sens que l'appareil recueille de manière continue la mesure de débit du gaz circulant dans la branche inspiratoire du circuit patient et actualise en permanence le débit de NO à délivrer afin de respecter la posologie désirée. Toutefois, dans certaines conditions, l'appareil de délivrance peut opérer une administration sous forme de débit pulsé, discontinu, par exemple en cas de faibles débits issus du ventilateur mécanique.

A des fins sécuritaires, un appareil de délivrance de NO dispose généralement d'une ligne de prélèvement de gaz permettant de prélever une portion du gaz inspiré par le patient, typiquement 100ml/min environ, et préférentiellement raccordée au niveau de la pièce Y du circuit patient, afin de l'analyser et de déterminer les teneurs en gaz dans le circuit patient, notamment :
- la concentration en NO qui doit être la plus proche possible de la posologie désirée,
- la concentration en oxygène (O₂) qui est variable et dépend des réglages effectués d'une part sur le ventilateur mécanique (enrichissement en O₂) et de la posologie désirée. En effet, une forte posologie associée à un faible enrichissement en oxygène peut en effet se traduire par un mélange dit hypoxique pouvant entraîner des dommages importants au patient.
- la concentration en dioxyde d'azote (NO₂) qui doit être la plus faible possible et détectée à de très faibles concentrations, typiquement de 0 à 5 ppm en volume. En effet, le NO₂, qui résulte de la réaction entre le NO et l'O₂ est un gaz extrêmement toxique pouvant entraîner de graves lésions chez le patient, dès des concentrations faibles de l'ordre de quelques ppm en volume.

Par ailleurs, le document US-A-2006/207594 enseigne un dispositif d'administration de mélange NO/N₂ d'architecture similaire à celle de US-A-5,558,083 puisqu'il comprend une ligne d'injection de NO délivrant du NO dans un circuit patient d'un ventilateur, lequel circuit patient est en liaison avec une ligne de mesure de concentration servant à mesurer les teneurs en gaz dans le circuit patient.

Le document EP-A-872254 décrit un analyseur utilisable pour mesurer les teneurs en les différents gaz susmentionnés, c'est-à-dire NO, NO₂ et O₂, comportant une pompe qui vient prélever du gaz à analyser en pièce Y du circuit patient. La pompe, qui est située en aval des éléments ou capteurs de mesure, permet de faire mettre le gaz au contact des capteurs pour réaliser la mesure. Le gaz utilisé pour les mesures est ensuite évacué vers l'atmosphère ambiante.

Dans cet appareil, les éléments de mesure de concentration mettent en oeuvre une technologie de type électrochimique. Or, cette technologie n'est pas exempte de contrainte car ce type de capteurs souffre d'une dérive et nécessite une calibration périodique.

Ainsi, l'étalonnage à « zéro » des capteurs consiste à présenter aux éléments de mesure un mélange gazeux de référence dépourvu de NO et de NO₂ afin de déterminer leur réponse nominale au repos, c'est-à-dire sans excitation découlant de la présence de ces gaz. Ceci est généralement réalisé en prélevant l'air ambiant où les teneurs de NO et NO₂ sont faibles car fortement diluées. Pour ce faire, le dispositif met en oeuvre une électrovanne qui, en fonctionnement normal, assure la liaison entre le point de prélèvement du gaz et la pompe mais qui, dans le cadre d'un étalonnage de zéro des capteurs, est commutée pour assurer une liaison entre l'air ambiant et la pompe qui fournit alors aux capteurs un mélange gazeux sans NO, ni NO₂.

Par ailleurs, le gain des capteurs qui consiste à présenter aux éléments de mesure un mélange gazeux calibré de NO et NO₂ afin de déterminer leur réponse à une concentration en NO ou NO₂ donnée. Ceci se fait à intervalles réguliers, en général tous les mois.

Or, cette procédure peut être assez longue, c'est-à-dire de l'ordre de 20 minutes, et nécessite par ailleurs des moyens conséquents, en particulier de bouteilles étalonnées à une concentration donnée, typiquement une bouteille de NO dosé à 40 ppmv et une bouteille de NO₂ dosé à 10 ppmv. La phase de calibration du capteur de NO consiste alors à connecter la bouteille de NO à 40 ppmv à l'entrée de l'analyseur, régler un débit donné de NO au moyen d'un débitmètre et injecter ce débit pendant un temps suffisant jusqu'à l'obtention d'un signal stable aux bornes du capteur. La technologie électrochimique étant relativement lente, plusieurs dizaines de secondes peuvent être nécessaires. On procède de la même façon avec la bouteille de NO₂.

On comprendra aisément que la fréquence préconisée de réalisation de ces calibrations ainsi que le temps consacré à la réalisation des procédures impose au personnel hospitalier une charge de travail non négligeable et immobilise régulièrement les appareils.

Outre l'aspect humain, il est à ajouter que l'emploi de bouteilles calibrées de NO et de NO₂ représente un coût d'exploitation non négligeable.

Enfin, chacune des bouteilles étalon dispose d'une date de péremption au delà de laquelle la concentration indiquée sur la bouteille pourrait ne pas être respectée, entraînant par là une erreur d'étalonnage. Il convient donc à chaque fois, pour des raisons de sécurité, de s'assurer que les mélanges gazeux étalons sont toujours utilisables ou, à défaut, les remplacer ce qui a un coût non négligeable.

Partant de là, le problème qui se pose est de remédier à la problématique de calibration périodique en proposant un appareil de délivrance de gaz, en particulier de mélanges NO/N₂, qui intègre des éléments de mesure de concentration des gaz, c'est-à-dire un dispositif analyseur de gaz, permettant d'espacer la fréquence de calibration périodique et de simplifier cette procédure, lequel ne nécessite pas l'usage de bouteilles étalon contenant du NO ou du NO₂ en teneurs étalonnées.

La solution de l'invention est un appareil de distribution de gaz, en particulier de mélange NO/azote, comprenant :
- une ligne d'injection de gaz comprenant une entrée de gaz et une sortie de gaz, et permettant de véhiculer un gaz depuis l'entrée de gaz jusqu'à la sortie de gaz, c'est-à-dire que la ligne d'injection de gaz est conçue pour et apte à véhiculer du gaz depuis l'entrée jusqu'à la sortie de gaz,
- une ligne d'analyse de gaz reliant fluidiquement un dispositif analyseur de gaz à une entrée de prélèvement gazeux,
- un dispositif de pilotage commandant le passage de gaz dans la ligne d'injection de gaz et le prélèvement de gaz par la ligne d'analyse de gaz,
caractérisé en ce qu'il comporte, en outre, une ligne de calibration raccordée fluidiquement à la ligne d'injection de gaz et à la ligne d'analyse de gaz de manière à permettre un passage direct de gaz de la ligne d'injection de gaz à la ligne d'analyse de gaz.
- la ligne d'injection de gaz comprend une première électrovanne commandée par le dispositif de pilotage,
- la ligne de calibration comprend une deuxième électrovanne commandée par le dispositif de pilotage,
- la ligne d'analyse de gaz comprend une quatrième électrovanne, située entre l'entrée de prélèvement et la deuxième électrovanne, et
- le dispositif analyseur de gaz est relié à la ligne d'analyse de gaz, entre lesdites deuxième et quatrième électrovannes.

Selon le cas, l'appareil de l'invention peut comprendre l'une ou plusieurs des caractéristiques techniques suivantes :
- il comporte, en outre, une ligne de secours reliée fluidiquement à la ligne d'injection de gaz, en amont et en aval de la première électrovanne agencée sur ladite ligne d'injection de gaz, de préférence ladite ligne de secours comprend une troisième électrovanne commandée par le dispositif de pilotage.
- il comporte, en outre, une électrovanne multi-voie agencée sur la ligne d'injection de gaz entre la première électrovanne et l'entrée de gaz, et à laquelle est raccordée la ligne de calibration et/ou la ligne de secours.
- l'électrovanne multi-voie est une électrovanne 3-voies.
- l'électrovanne multi-voie est commandée par le dispositif de pilotage.
- le dispositif de pilotage comprend une carte électronique à microprocesseur.
- le dispositif analyseur de gaz comprend plusieurs éléments de mesure permettant de mesurer la concentration en NO, NO₂ et O₂, lesdits éléments de mesure étant reliés au dispositif de pilotage et transformant chacun une concentration en gaz en un signal électrique envoyé à et exploité par le dispositif de pilotage.
- le dispositif analyseur de gaz comprend un premier élément de mesure permettant de mesurer la concentration en NO, un deuxième élément de mesure permettant de mesurer simultanément la concentration en NO et NO₂, et un troisième élément de mesure permettant de mesurer la concentration en O₂.
- le dispositif analyseur de gaz comprend plusieurs éléments de mesure permettant de mesurer la concentration en NO, NO₂ et O₂, lesdits éléments de mesure étant agencés un tronçon venant se raccorder fluidiquement à la ligne de prélèvement.
- ledit tronçon constitue une ligne de mise à l'atmosphère ambiante en étant relié par son autre extrémité à l'atmosphère.
- les premier, deuxième et troisième éléments de mesure sont agencés successivement sur la ligne de mise à l'atmosphère ambiante de manière à ce que le flux de gaz à analyser traverse d'abord le premier élément, puis le deuxième élément et finalement le troisième élément de mesure.
- le dispositif analyseur de gaz comprend un élément de mesure à plusieurs canaux de détection afin de mesurer, de manière simultanée, la concentration en NO et en NO₂.
- le dispositif analyseur de gaz comprend un ou plusieurs éléments de mesure à infrarouge. Il est à noter cependant que d'autres types de capteurs peuvent également parfaitement convenir dès lors qu'ils permettent d'assurer les mesures en NO, NO₂ et O₂ désirées, notamment des capteurs électrochimiques ou reposant sur le principe de photo ionisation.
- ladite quatrième électrovanne étant une électrovanne à 3 voies dont une voie est reliée à l'atmosphère.
- la ligne de prélèvement comprend en outre une pompe et un capteur de débit connectés au dispositif de pilotage.
- la ligne d'injection de gaz comprend en outre un capteur de pression connecté au dispositif de pilotage.
- la ligne d'injection de gaz, la ligne d'analyse de gaz et le dispositif de pilotage et la ligne de calibration sont agencés au sein d'un boitier unique.

Par ailleurs, l'invention concerne aussi une installation de distribution d'un gaz contenant du NO comprenant :
- un ventilateur pour délivrer un gaz respiratoire contenant de l'oxygène,
- un circuit patient reliant le ventilateur à un patient,
- un appareil selon l'invention connecté fluidiquement audit circuit patient via la ligne d'injection de gaz et la ligne de prélèvement, et
- une source de NO gazeux alimentant la ligne d'injection de gaz de l'appareil avec un gaz contenant du NO.
- la source de NO gazeux, telle une bouteille de gaz, alimentant la ligne d'injection de gaz de l'appareil contient un mélange gazeux formé d'azote et de 1 à 1000 ppmv de NO.

L'invention va maintenant être décrite plus en détail en rapport avec les Figures annexées parmi lesquelles :
- la Figure 1 est une représentation schématique d'un appareil de distribution de NO selon l'art antérieur avec ligne d'injection de NO et ligne de prélèvement du gaz inhalé totalement indépendantes pneumatiquement l'une de l'autre ;
- la Figure 2 est une représentation schématique d'un mode de réalisation d'un appareil de distribution de NO selon l'invention avec liaison pneumatique entre les lignes d'injection de NO et de prélèvement du gaz inhalé ; et
- la Figure 3 est une description détaillée du mode de réalisation de l'appareil de la Figure 2.

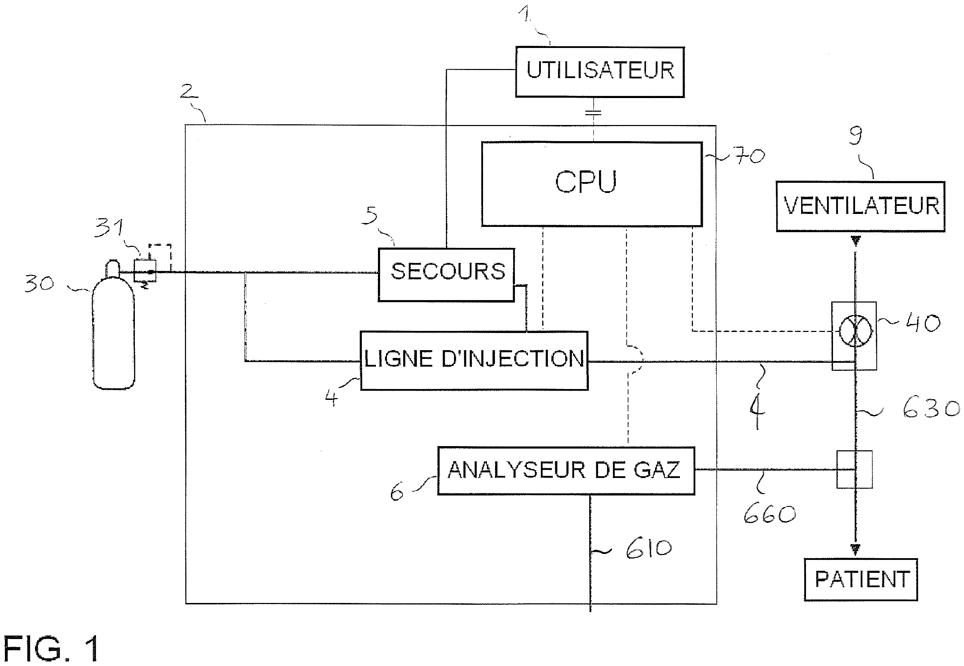

La Figure 1 représente un appareil 2 de distribution de NO selon l'art antérieur avec ligne d'injection 4 de NO et ligne de prélèvement 660 du gaz inhalé totalement pneumatiquement indépendantes l'une de l'autre, c'est-à-dire non reliées directement l'une à l'autre.

Plus précisément, un tel appareil 2 enrichit en NO gazeux, un mélange gazeux respiratoire, par exemple de l'air ou un mélange O₂/N₂, provenant par exemple d'un ventilateur mécanique 9 selon une posologie déterminée par un utilisateur 1, typiquement un médecin ou similaire. La posologie désirée, habituellement comprise entre 10 et 80 ppm en volume de NO, est réglée sur l'appareil 2 par l'utilisateur 1 au moyen d'organes de réglage, tels que des boutons de réglages ou bien une interface homme/machine comprenant un écran tactile par exemple.

Afin de délivrer la posologie souhaitée, le dispositif 2 est généralement alimenté par une ou plusieurs bouteilles 30 contenant une concentration donnée de NO, variant généralement entre 250 et 800 ppmv, le reste étant un gaz inerte, tel de l'azote. Par exemple les mélanges NO/N₂ à 225 ou à 450 ppmv sont disponibles auprès de la société Air Liquide Santé.

Cette alimentation par une bouteille de gaz 30 peut être directe sur certains dispositifs en ce sens que ces derniers sont directement soumis à la pression de la bouteille 30, typiquement environ 150 bars, ou bien alimentés de manière indirecte en ce sens qu'un régulateur de pression 31, intégré à la bouteille 30 par exemple, abaisse la pression du gaz délivré par cette dernière à une pression plus faible, typiquement 3,5 bar.

Comme enseigné par le document US-A-5,558,083, l'enrichissement en NO opéré par le dispositif 2 dépend alors de la posologie réglée par l'utilisateur 1, du débit issu du ventilateur mécanique 9 et mesuré par le module d'injection 40, et de la concentration en NO de la bouteille du système d'alimentation en gaz

Pour assurer la posologie souhaitée, l'appareil 2 met en oeuvre une ligne d'injection 4 qui assure la délivrance de la quantité appropriée de NO.

Par ailleurs, est également prévu une ligne de secours 5 qui est utilisée lors d'une défaillance majeure du dispositif 2 et vise à ne pas interrompre brutalement la thérapie par NO inhalé, en cas de défaillance de la ligne d'injection 4, car une telle interruption pourrait avoir des conséquences cliniques importantes pour le patient.

Une telle ligne de secours 5 se présente sous différentes forme sur les dispositifs connus. Elle peut être agencée aussi bien à l'extérieur du dispositif 2 et comprendre une bouteille de secours associée à des moyens de détente de la pression et de réglage d'un débit fixe de NO/N₂, ou bien être située à l'intérieur même du dispositif 2 en étant formée d'une ligne pneumatique 5 séparée venant se raccorder en deux sites de la ligne d'injection 4, comme illustré en Figure 1. La commutation en position de secours du dispositif 2 se fait manuellement dans les appareils existants.

En outre, l'appareil comprend aussi un analyseur de gaz 6 qui détermine les concentrations en NO, NO₂ et O₂ à partir d'échantillons de gaz prélevés dans le circuit patient via une ligne d'analyse 660 qui alimente l'analyseur 6 en lesdits échantillons gaz.

L'association de ces 3 sous-ensembles, à savoir la ligne d'injection 4, la ligne de secours 5 et l'analyseur de gaz 6, est au coeur des dispositifs de délivrance de NO actuels.

Avec ce type d'appareil 2 de l'art antérieur, la calibration des éléments de mesure, c'est-à-dire des capteurs, de l'analyseur de gaz 6 met en oeuvre des mélanges gazeux calibrés de NO et de NO₂, c'est-à-dire des bouteilles étalonnées à une concentration donnée, typiquement une bouteille de NO dosé à 40 ppmv et une bouteille de NO₂ dosé à 10 ppmv, et doit être faite à intervalles réguliers, en général tous les mois. Ces bouteilles étalonnées à une concentration donnée sont raccordées directement à la ligne 660.

Les gaz ayant servi à la calibration sont ensuite envoyés à l'atmosphère via une ligne de mise à l'atmosphère ambiante 610, c'est-à-dire un tronçon de passage de gaz en communication fluidique avec la ligne de prélèvement 660 et sur laquelle sont agencés les prises de mesure des capteurs de l'analyseur 6.

Comme déjà mentionné, la fréquence préconisée de réalisation de ces calibrations ainsi que le temps consacré à la réalisation des procédures impose au personnel hospitalier une charge de travail non négligeable et immobilise par ailleurs régulièrement les appareils. Ceci n'est pas pratique.

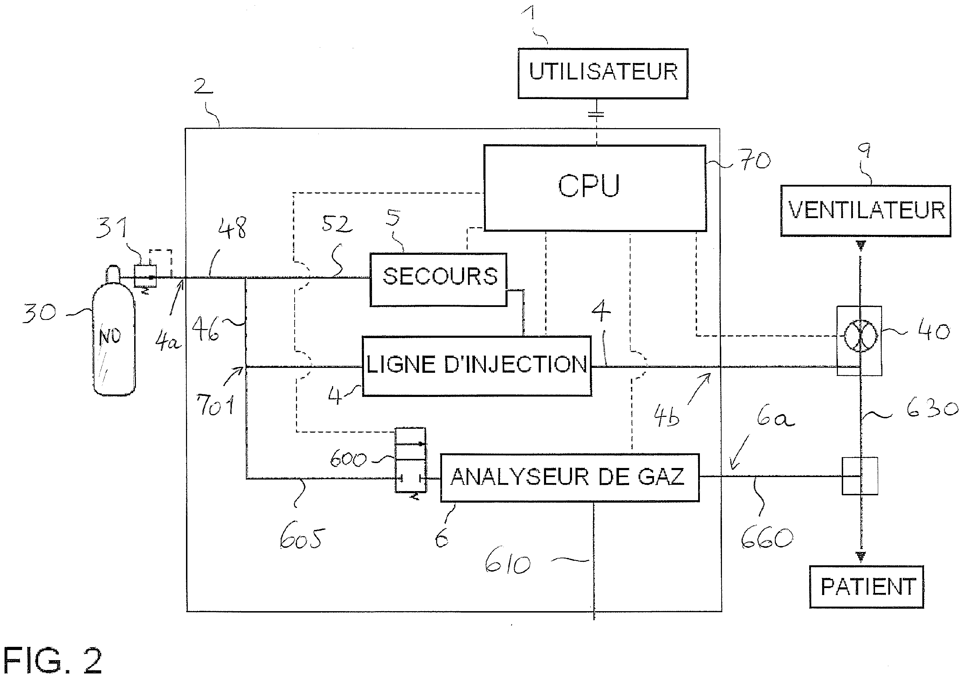

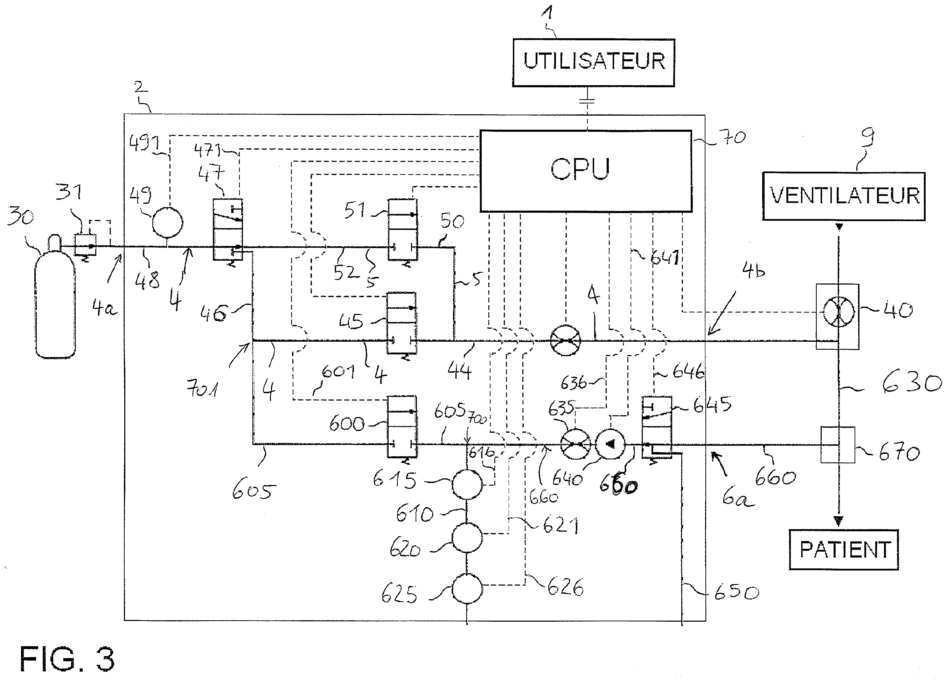

La Figure 2 est une représentation schématique d'un mode de réalisation d'un appareil 2 de distribution de NO/N₂ selon l'invention comprenant une liaison pneumatique supplémentaire, appelée ligne de calibration 605, entre les lignes d'injection 4 de NO et de prélèvement 660 du gaz inhalé, permettant de palier les inconvénients susmentionnés et de réaliser efficacement et aisément les calibrations des éléments de mesure 615, 620, 625 (cf. Figure 3), c'est-à-dire des capteurs, de l'analyseur de gaz 6.

La ligne de calibration 605 est raccordée fluidiquement (en 701) à la ligne d'injection de gaz (4) et (en 700) à la ligne d'analyse de gaz 660 de manière à permettre un passage direct de gaz de la ligne d'injection de gaz 4 à la ligne d'analyse de gaz 660, puis au tronçon 610 portant les éléments de mesure et d'analyse 615, 620, 625, comme détaillé ci-après..

L'agencement de cette ligne de calibration 605 permet d'assurer une communication pneumatique, c'est-à-dire le passage direct de gaz à base de NO, entre les lignes d'injection 4, de secours 5 et l'analyseur de gaz 6, comme expliqué ci-après.

Comme détaillé sur la Figure 3, la circulation de gaz au sein de la ligne de prélèvement 660 est contrôlée par une électrovanne 645 commandée par un dispositif de pilotage 70, tel une unité à microprocesseur (CPU), qui permet notamment de réaliser une calibration automatique des différents éléments de mesure 615, 620, 625 de l'analyseur 6 de gaz ; de détecter automatiquement la concentration en NO du système d'alimentation en gaz du dispositif ; et de permettre périodiquement une purge du système d'alimentation en gaz via la ligne de mise à l'atmosphère 610 ambiante.

Par ailleurs, un capteur de pression 49 est agencé sur le tronçon 48 de la ligne d'injection 4, lequel tronçon 48 est situé au niveau de l'entrée 4a de gaz du dispositif 2, afin de mesurer la pression gazeuse fournie par le système d'alimentation en gaz, comprenant la bouteille 30 et le détendeur 31, typiquement une pression de l'ordre de 3,5 bar.

La valeur de pression déterminée par le capteur 49 est retournée au dispositif de pilotage 70 comprenant une ou plusieurs cartes de commande, par le biais d'une liaison électrique 491, ce qui permet de s'assurer que le dispositif 2 est correctement alimenté en gaz contenant du NO.

En condition normale de fonctionnement, c'est-à-dire sans recours à la ligne de secours 5, une électrovanne à 3 voies 47 est pilotée grâce à la liaison 471 par le dispositif de pilotage 70 de telle manière à garantir une liaison entre le tronçon d'admission des gaz 48 et une première électrovanne 45, appelée électrovanne de dosage, située sur le tronçon de transmission 46 de la ligne d'injection 4, entre l'électrovanne à 3-voies et la sortie 4b de la ligne d'injection 4.

Cette position de l'électrovanne à 3 voies 47 permet également de mettre en relation le tronçon d'admission des gaz 48 et l'électrovanne de calibration 600 par l'intermédiaire du tronçon de transmission 46 de la ligne d'injection 4 et la ligne de calibration 605 qui est raccordée fluidiquement à la ligne d'injection de gaz 4 et à la ligne d'analyse de gaz 610, via le tronçon 605, de manière à permettre un passage direct de gaz contenant du NO de la ligne d'injection de gaz 4 à la ligne d'analyse de gaz 660, en particulier au tronçon 610, comprenant le dispositif d'analyse 6. Cette disposition est détaillée ci-après.

L'électrovanne de dosage 45 est ainsi alimentée en gaz provenant de la source de NO 30 et assure de manière classique, le dosage des gaz dans le tronçon d'injection 44 de la ligne d'injection 4 en fonction de la posologie réglée par l'utilisateur 1, du débit gazeux issu du ventilateur mécanique 9 et mesuré par le module d'injection 40 et de la concentration en NO de la bouteille 30 du système d'alimentation en gaz.

En condition normale de fonctionnement, l'électrovanne 3 voies 47 est pilotée par le dispositif de pilotage 70 de manière à ce que l'électrovanne de secours 51 ne soit pas alimentée. Seule une condition de défaillance majeure du dispositif, telle que la perte de toute commande électrique, déclenche le système de secours de sorte qu'un débit de NO traverse l'électrovanne de secours 51 afin de continuer à administrer au patient une quantité donnée de NO.

Par ailleurs, lorsque le dispositif 2 administre une posologie donnée de NO au patient, l'électrovanne 600 de calibration est commandée en position fermée par la carte de commande du dispositif de pilotage 70 via la liaison électrique 601 de sorte que la ligne de calibration 605 ne soit pas alimentée par le mélange gazeux NO/N₂ provenant du système 30 d'alimentation en gaz.

Une quatrième électrovanne 645, appelée électrovanne de zéro, agencée sur la ligne de prélèvement 660 entre l'entrée de prélèvement 6a et l'électrovanne 600 de calibration, est également commandée par la carte de commande du dispositif de pilotage 70, via une liaison électrique 646, de telle manière que la communication fluidique au sein de la ligne de prélèvement 660 soit permise et assurée, depuis l'entrée 6a et jusqu'à l'analyseur de gaz 6.

La quatrième électrovanne (645) est en fait une électrovanne à 3 voies ayant une voie 650 reliée à l'atmosphère et ses deux autres voies reliées à la ligne de prélèvement 660.

La ligne de prélèvement 660 est connectée au plus proche du patient par le biais d'un raccord 670, par exemple une pièce en Y du circuit patient 630, afin que les gaz analysés soient représentatifs de ce qui est inhalé par le patient.

La ligne de prélèvement de gaz 660 comporte en outre une pompe 640 aspirante et un capteur de débit 635 agencés entre la quatrième électrovanne 645 et l'électrovanne 600 de calibration.

Le prélèvement de gaz est rendu possible grâce à la pompe 640 qui est commandée par la carte de commande du dispositif de pilotage 70 via une liaison électrique 641 et qui aspire le gaz sous un débit donné, et est contrôlé au moyen du capteur de débit 635 coopérant avec la carte de commande du dispositif de pilotage 70 pour récupérer, via la liaison 636, la (les) valeur de débit au sein de la ligne 660.

Il s'agit dès lors d'un système régulé puisque la carte de commande du dispositif de pilotage 70 pilote la pompe 640 afin que le débit prélevé et mesuré par le capteur de débit 635 reste constant. On notera que le capteur de débit 635 peut être directement intégré dans la pompe et renvoyé par celle-ci via la liaison 641 à la carte de commande du dispositif de pilotage 70 comme un élément de mesure

Le débit ainsi prélevé, typiquement entre 50 et 200 ml/min, est ensuite analysé par un ensemble d'éléments de mesure 615, 620, 625 de l'analyseur 6, avant d'être évacué de l'appareil et rejeté, par exemple à l'atmosphère à travers la ligne de mise à l'atmosphère 610, encore appelée canal d'analyse.

Cette analyse du gaz prélevé dans le circuit patient 630 permet de fournir des informations sur les concentrations en gaz envoyées vers le patient, c'est-à-dire les concentrations en NO, NO₂ et O₂. Ces informations sont par exemple affichées sur un écran et/ou mémorisées.

L'analyseur de gaz 6 comporte plusieurs capteurs ou éléments de mesure 615, 620 et 625 permettant de mesurer la teneur en NO, NO₂ et O₂ dans le flux gazeux prélevé.

L'élément de mesure 620 est par exemple un capteur de type à infrarouge comprenant un émetteur et un détecteur, par exemple le capteur commercialisé par Micro-Hybrid Gmbh. Toutefois, d'autres types de capteurs peuvent être utilisés.

L'élément de mesure 620 comprend plusieurs canaux de détection afin de mesurer de manière simultanée, la concentration en NO et en NO₂ et de pouvoir alors envoyer à la carte de commande du dispositif 70, les concentrations en NO et NO₂ mesurées, via une liaison 621. Ces concentrations peuvent être affichées sur un écran et/ou mémorisées.

Outre les concentrations en NO et NO₂ il est également nécessaire de mesurer celle en O₂. Ceci est réalisé par le biais de l'élément de mesure 625, par exemple le capteur d'oxygène commercialisé par S4MS sous la référence SMSI 02 Sensor, qui envoie la concentration en O₂ à la carte de commande du dispositif 70 via la liaison 626.

Un élément de mesure supplémentaire 615 est spécialement dimensionné pour mesurer de fortes concentrations en NO telles que celles présentes dans la bouteille d'alimentation 30.

Pour tout système mettant en oeuvre des éléments de mesure, une utilisation prolongée peut entraîner une dérive dans l'appréciation des concentrations en gaz, à savoir NO, NO₂ et O₂. Or, utiliser un capteur à infra rouge 620 par rapport à une technologie plus classique, électrochimique notamment, permet de limiter la fréquence de réalisation des opérations de calibration en NO et NO₂ en passant d'une préconisation de calibration mensuelle à annuelle. Néanmoins, il est également possible d'utiliser un capteur électrochimique si la fréquence de calibration n'est pas problématique.

La procédure de calibration et d'étalonnage de l'analyseur 6 se fait de la manière suivante.

En supposant que l'appareil 2 est en condition normale de fonctionnement, c'est-à-dire que l'électrovanne à 3 voies d'admission 47 garantit une liaison entre le tronçon 48 d'admission des gaz et l'électrovanne de calibration 600, via la ligne de calibration 605, il est possible de piloter cette électrovanne de calibration 600 par le biais de la carte de commande du dispositif 70, via la liaison électrique 601, afin de la commuter en position ouverte pour que le NO issu du système d'alimentation en gaz puisse circuler dans la ligne de calibration 605 puis dans la ligne de mise à l'atmosphère 610, encore appelée canal d'analyse.

En parallèle, la carte de commande du dispositif de pilotage 70 commande, via la liaison 641, la pompe 640 de manière à la stopper et donc à arrêter les prélèvements de gaz issu du circuit patient 630.

Dès lors, le gaz circulant dans la ligne de calibration 605 et dans la ligne de mise à l'atmosphère 610, donc soumis à l'analyse par les éléments 615, 620, 625 de l'analyseur 6, est identique au gaz contenu dans la bouteille 30 du système d'alimentation en gaz.

Or, la concentration d'une telle bouteille 30 est connue, par exemple 450 ppmv, avec une précision de concentration en NO de l'ordre de 1% en volume, de sorte que la valeur réelle oscille entre 445 et 455 ppmv dans l'exemple précédent, ce qui est largement suffisant pour entamer une procédure fiable d'étalonnage.

L'élément de mesure 615, également basé sur une technologie infrarouge ou autre, est à cet effet spécialement dimensionné pour détecter des niveaux de concentration en NO élevés, c'est-à-dire jusqu'à 1000 ppmv. Il est alors possible, si nécessaire, de calibrer ce capteur par rapport à la concentration connue en NO de la bouteille 30. La carte de commande du dispositif 70 recueille alors la valeur retournée par cet élément de mesure 615 via la liaison électrique 616 pour en déduire un coefficient de calibration.

Or, il apparaît également que le mélange issu de la bouteille 30 est dépourvu d'oxygène et passe devant l'élément de mesure 625 détectant justement les niveaux d'oxygène. Il est alors possible dans le même temps de réaliser le « zéro » de ce capteur, c'est à dire d'exploiter la valeur retournée via la liaison électrique 626 par la carte de commande 70.

On notera que cette procédure peut également être utilisée pour détecter et indiquer à l'utilisateur la concentration de la bouteille connectée au dispositif 30 pour le cas où des bouteilles avec des teneurs en NO différentes seraient utilisées.

D'autre part, il apparaît de façon évidente qu'une telle injection de NO pour réaliser cette première phase de calibration des éléments de mesure 615 et 625, non influencés par une éventuelle concentration indésirable de NO₂ peut être avantageusement utilisée pour purger le système d'admission des gaz lorsque cela est nécessaire.

Avec le dispositif de l'invention, il est également possible d'opérer la calibration à « zéro » de l'analyseur 6 pour ce qui concerne le NO et le NO₂.

Pour ce faire, on pilote l'électrovanne de calibration 600 (électrovanne dite « de zéro ») au moyen de la carte de commande du dispositif 70, via la liaison 601, de sorte que celle-ci soit commutée en position fermée, ce qui interrompt le flux de NO circulant dans la ligne de calibration 605.

Ensuite, on commute la quatrième électrovanne à 3 voies 645 par le biais de la carte de commande du dispositif de pilotage 70, via la liaison électrique 646, de manière à ce que celle-ci garantisse une liaison fluidique entre le canal d'atmosphère 650 et la portion de ligne de prélèvement 660 située entre les électrovannes 654 et 600.

Le débit gazeux issu du canal 650 et aspiré par la pompe 640, qui est représentatif de l'air ambiant, est exempt ou quasi exempt de NO et NO₂ et à la concentration en O₂ de l'air, c'est-à-dire 21% en volume.

Dès lors, le débit gazeux traversant les éléments de mesure 615, 620, 625 permet de réaliser différentes actions sur ces éléments, à savoir :
- Le zéro sur les capteurs 615 et 620 mesurant respectivement les concentrations de NO et de NO/NO₂. Ces éléments de mesure en renvoyant les informations de zéro par les liaisons 616 et 621 permettent à la carte de commande 70 de les exploiter.
- le gain du capteur 625 d'O₂. En effet, celui-ci aura été précédemment conditionné pour réaliser dans de bonnes conditions son zéro (le point à 0% d'O₂ via le mélange NO/N₂ issu de la source 30). L'air étant à 21% d'O₂, il est alors possible de réaliser un deuxième point d'étalonnage et en déterminer une nouvelle loi de mesure. La valeur retournée par le capteur d'O₂ 625, via la liaison électrique 626, est alors exploitée par la carte de commande du dispositif de pilotage 70.

Il est alors remarquable d'observer que le capteur d'O₂ 625 est alors calibré ainsi que l'élément de mesure 615. Le capteur de NO/NO₂ 620 est, quant à lui, déjà étalonné à zéro.

Ensuite, le cycle de calibration, en considérant toujours un fonctionnement normal, se termine de la manière suivante.

La pompe 640 continue à prélever de l'air ambiant libre de tout NO et NO₂. A l'inverse, l'électrovanne de calibration 600 est commutée en position ouverte et le débit circulant dans le canal d'analyse 610 est alors un mélange d'air ambiant prélevé par la pompe 635 et de NO à la concentration de la bouteille 30 amené par la ligne de calibration 605.

En ajustant le débit prélevé par la pompe 635 par l'intermédiaire de la carte de commande 70 de manière à ce que l'élément de mesure 615, parfaitement calibré retourne une valeur à la carte de commande 70 telle que celle-ci soit égale à 100 ppmv (plus le débit envoyé par la pompe est élevé et plus le NO est dilué), il est alors possible de calibrer l'élément de mesure 620. En effet, la dérive qui affecte le capteur à infrarouge est essentiellement le fait de l'atténuation de la source infra rouge. Puisque cette source infrarouge alimente simultanément les 2 canaux de mesure de NO et NO₂ de cet élément de mesure 620, une calibration du canal NO induit automatiquement une calibration du canal NO₂. Or, le fait d'ajuster la teneur du mélange à une concentration de 100 ppmv en NO permet de calibrer le canal NO de l'élément de mesure 620 et par extension le canal NO₂ du même capteur. De la même manière, la valeur retournée par le capteur à la carte de commande 70, via la liaison électrique 621, est utilement exploitée pour rendre la mesure plus précise.

Il apparaît alors qu'à l'issue de ces différentes séquences de calibration, tous les éléments de mesure 615, 620 et 625 de l'analyseur 6 sont parfaitement calibrés et permettent l'affichage des concentrations en NO, NO₂ et O₂ de manière fiable.

Cette calibration ou étalonnage ne nécessité aucune action et ni aucun moyen extérieurs, ce qui soulage le personnel hospitalier et fiabilise les calibrations des éléments de mesure.

Ces différentes séquences consistant à ouvrir ou fermer l'électrovanne de calibration 600 et provoquer l'arrêt ou le contrôle de la pompe 640 permettent ainsi de réaliser de manière organisée la calibration de l'ensemble des éléments de mesure détectant les concentrations de gaz.

La calibration peut être engagée à tout moment, à savoir lorsque le dispositif est allumé mais ne délivre pas de NO, par exemple à son démarrage, ou bien à l'occasion d'une procédure décidée par l'utilisateur 1 via l'interface de contrôle, que l'appareil soit à l'arrêt ou bien en cours de délivrance.

En effet, dans la disposition actuelle l'électrovanne 3 voies assure la liaison entre le tronçon d'admission des gaz 48 et l'électrovanne de calibration 600 via le tronçon de transmission 46 mais aussi avec l'électrovanne de dosage 45 via le même tronçon 46. Cette électrovanne 3 peut ainsi délivrer la posologie souhaitée alors même qu'une procédure de calibration des éléments de mesure est engagée puisque le flux gazeux contenant le NO pourra non seulement être envoyé vers le circuit patient 630, via la ligne d'injection 4, mais aussi être simultanément envoyer vers l'analyseur 6 via la ligne de calibration 605 reliée au tronçon 46.

Il convient de noter que le dispositif de la présente invention permet également de remplir avantageusement des fonctions supplémentaires, tel que purge du système et détection automatique de la concentration en NO d'une bouteille connectée au dispositif.

L'appareil et l'installation de l'invention sont particulièrement bien adaptés à une mise en oeuvre dans le cadre d'une méthode de traitement thérapeutique de l'adulte ou de l'enfant, notamment des nouveaux-nés, souffrant d'hypertension artérielle pulmonaire que l'on traite par administration par voie inhalée d'oxyde nitrique ou monoxyde d'azote permettant de dilater les vaisseaux sanguins des poumons et augmenter l'oxygénation du sang en favorisant les échanges gazeux pulmonaires chez ces patients.

## Revendications

1. Appareil de distribution de gaz comprenant :
- une ligne d'injection de gaz (4) comprenant une entrée de gaz (4a) et une sortie de gaz (4b), et permettant de véhiculer un gaz depuis l'entrée de gaz (4a) jusqu'à la sortie de gaz (4b),
- une ligne d'analyse de gaz (660) reliant fluidiquement un dispositif analyseur de gaz (6) à une entrée de prélèvement gazeux (6a),
- un dispositif de pilotage (70) commandant le passage de gaz dans la ligne d'injection de gaz (4) et le prélèvement de gaz par la ligne d'analyse de gaz (660),
**caractérisé en ce que** :
- il comporte, en outre, une ligne de calibration (605) raccordée fluidiquement (701 ; 700) à la ligne d'injection de gaz (4) et à la ligne d'analyse de gaz (660) de manière à permettre un passage direct de gaz de la ligne d'injection de gaz (4) à la ligne d'analyse de gaz (660),
- la ligne d'injection de gaz (4) comprend une première électrovanne (45) commandée par le dispositif de pilotage (70),
- la ligne de calibration (605) comprend une deuxième électrovanne (600) commandée par le dispositif de pilotage (70),
- la ligne d'analyse de gaz (660) comprend une quatrième électrovanne (645), située entre l'entrée de prélèvement (6a) et la deuxième électrovanne (600), et
- le dispositif analyseur de gaz (6) est relié à la ligne d'analyse de gaz (660), entre lesdites deuxième (600) et quatrième électrovannes (645).

2. Appareil selon la revendication précédente, **caractérisé en ce qu'**il comporte, en outre, une ligne de secours (5) reliée fluidiquement à la ligne d'injection de gaz (4), en amont et en aval de la première électrovanne (45) agencée sur ladite ligne d'injection de gaz (4).

3. Appareil selon la revendication précédente, **caractérisé en ce que** ladite ligne de secours (5) comprend une troisième électrovanne (51) commandée par le dispositif de pilotage (70).

4. Appareil selon l'une des revendications précédentes, **caractérisé en ce qu'**il comporte, en outre, une électrovanne multi-voie (47) agencée sur la ligne d'injection de gaz (4) entre la première électrovanne (45) et l'entrée de gaz (4a), et à laquelle est raccordée la ligne de calibration (605) et/ou la ligne de secours (5).

5. Appareil selon l'une des revendications précédentes, **caractérisé en ce que** l'électrovanne multi-voie (47) est une électrovanne 3-voies, de préférence l'électrovanne multi-voie (47) est commandée par le dispositif de pilotage (70).

6. Appareil selon l'une des revendications précédentes, **caractérisé en ce que** le dispositif de pilotage (70) comprend une carte électronique à microprocesseur.

7. Appareil selon l'une des revendications précédentes, **caractérisé en ce que** le dispositif analyseur de gaz (6) comprend plusieurs éléments de mesure (615, 620, 625) permettant de mesurer la concentration en NO, NO₂ et O₂, lesdits éléments de mesure (615, 620, 625) étant reliés au dispositif de pilotage (70), de préférence le dispositif analyseur de gaz (6) comprend un premier élément de mesure (615) permettant de mesurer la concentration en NO, un deuxième élément de mesure (620) permettant de mesurer simultanément les concentrations en NO et en NO₂, et un troisième élément de mesure (625) permettant de mesurer la concentration en O₂.

8. Appareil selon l'une des revendications précédentes, **caractérisé en ce que** le dispositif analyseur de gaz (6) comprend plusieurs éléments de mesure (615, 620, 625) permettant de mesurer la concentration en NO, NO₂ et O₂, lesdits éléments de mesure (615, 620, 625) étant agencés un tronçon (610) venant se raccorder fluidiquement à la ligne de prélèvement (660), de préférence ledit tronçon (610) constitue une ligne de mise à l'atmosphère ambiante reliée par son autre extrémité à l'atmosphère.

9. Appareil selon l'une des revendications précédentes, **caractérisé en ce que** le dispositif analyseur de gaz (6) comprend un élément de mesure (620) à plusieurs canaux de détection afin de mesurer, de manière simultanée, la concentration en NO et en NO₂.

10. Appareil selon l'une des revendications précédentes, **caractérisé en ce que** le dispositif analyseur de gaz (6) comprend un ou plusieurs éléments de mesure (620) à infrarouge.

11. Appareil selon l'une des revendications précédentes, **caractérisé en ce que** ladite quatrième électrovanne (645) est une électrovanne à 3 voies dont une voie (650) est reliée à l'atmosphère.

12. Appareil selon l'une des revendications précédentes, **caractérisé en ce que** la ligne de prélèvement (660) comprend en outre une pompe (640) et un capteur de débit (635) connectés au dispositif de pilotage (70).

13. Appareil selon l'une des revendications précédentes, **caractérisé en ce que** la ligne d'injection de gaz (4) comprend en outre un capteur de pression (49) connecté au dispositif de pilotage (70).

14. Appareil selon l'une des revendications précédentes, **caractérisé en ce que** la ligne d'injection de gaz (4), la ligne d'analyse de gaz (660) et le dispositif de pilotage (70) et la ligne de calibration (605) sont agencés au sein d'un boitier unique.

15. Installation de distribution d'un gaz contenant du NO comprenant :
- un ventilateur (9) pour délivrer un gaz respiratoire contenant de l'oxygène,
- un circuit patient (630) reliant le ventilateur (9) à un patient,
- un appareil selon l'une des revendications précédentes connecté fluidiquement audit circuit patient (630) via la ligne d'injection de gaz (4) et la ligne de prélèvement (660), et
- une source de NO gazeux (30) alimentant la ligne d'injection de gaz (4) de l'appareil avec un gaz contenant du NO.

## Patentansprüche

1. Gasverteilungsgerät, umfassend:
- eine Gaseinspritzlinie (4), die einen Gaseinlass (4a) und einen Gasauslass (4b) umfasst und es ermöglicht, ein Gas vom Gaseinlass (4a) bis zum Gasauslass (4b) zu befördern,
- eine Gasanalyselinie (660), die eine Gasanalysiervorrichtung (6) strömungstechnisch mit einem Gasentnahmeeinlass (6a) verbindet,
- eine Steuervorrichtung (70), die den Durchlass von Gas in die Gaseinspritzlinie (4) und die Entnahme von Gas durch die Gasanalyselinie (660) steuert,
**dadurch gekennzeichnet, dass**:
- es des Weiteren eine Kalibrierlinie (605) aufweist, die strömungstechnisch (701; 700) an die Gaseinspritzlinie (4) und an die Gasanalyselinie (660) angeschlossen ist, um einen direkten Durchlass von Gas von der Gaseinspritzlinie (4) zur Gasanalyselinie (660) zu ermöglichen,
- die Gaseinspritzlinie (4) ein erstes Magnetventil (45) umfasst, das von der Steuervorrichtung (70) gesteuert wird,
- die Kalibrierlinie (605) ein zweites Magnetventil (600) umfasst, das von der Steuervorrichtung (70) gesteuert wird,
- die Gasanalyselinie (660) ein viertes Magnetventil (645) umfasst, das sich zwischen dem Entnahmeeinlass (6a) und dem zweiten Magnetventil (600) befindet, und
- die Gasanalysiervorrichtung (6) zwischen dem zweiten (600) und vierten Magnetventil (645) mit der Gasanalyselinie (660) verbunden ist.

2. Gerät nach dem vorstehenden Anspruch, **dadurch gekennzeichnet, dass** es des Weiteren eine Hilfslinie (5) aufweist, die vor und nach dem ersten Magnetventil (45), das an der Gaseinspritzlinie (4) angeordnet ist, strömungstechnisch mit der Gaseinspritzlinie (4) verbunden ist.

3. Gerät nach dem vorstehenden Anspruch, **dadurch gekennzeichnet, dass** die Hilfslinie (5) ein drittes Magnetventil (51) umfasst, das von der Steuervorrichtung (70) gesteuert wird.

4. Gerät nach einem der vorstehenden Ansprüche, **dadurch gekennzeichnet, dass** es des Weiteren ein Mehrwege-Magnetventil (47) aufweist, das zwischen dem ersten Magnetventil (45) und dem Gaseinlass (4a) an der Gaseinspritzlinie (4) angeordnet ist, und an das die Kalibrierlinie (605) und/oder die Hilfslinie (5) angeschlossen ist.

5. Gerät nach einem der vorstehenden Ansprüche, **dadurch gekennzeichnet, dass** es sich bei dem Mehrwege-Magnetventil (47) um ein 3-Wege-Magnetventil handelt, wobei das Mehrwege-Magnetventil (47) bevorzugt von der Steuervorrichtung (70) gesteuert wird.

6. Gerät nach einem der vorstehenden Ansprüche, **dadurch gekennzeichnet, dass** die Steuervorrichtung (70) eine Elektronikplatine mit Mikroprozessor umfasst.

7. Gerät nach einem der vorstehenden Ansprüche, **dadurch gekennzeichnet, dass** die Gasanalysiervorrichtung (6) mehrere Messelemente (615, 620, 625) umfasst, die das Messen der NO-, NO₂- und O₂-Konzentration ermöglichen, wobei die Messelemente (615, 620, 625) mit der Steuervorrichtung (70) verbunden sind, wobei die Gasanalysiervorrichtung (6) bevorzugt ein erstes Messelement (615) umfasst, das das Messen der NO-Konzentration ermöglicht, ein zweites Messelement (620), das das gleichzeitige Messen der NO- und NO₂-Konzentration ermöglicht, und ein drittes Messelement (625), das das Messen der O₂-Konzentration ermöglicht.

8. Gerät nach einem der vorstehenden Ansprüche, **dadurch gekennzeichnet, dass** die Gasanalysiervorrichtung (6) mehrere Messelemente (615, 620, 625) umfasst, die das Messen der NO-, NO₂- und O₂-Konzentration ermöglichen, wobei die Messelemente (615, 620, 625) an einem Teilabschnitt (610) angeordnet sind, der sich strömungstechnisch an die Entnahmelinie (660) anschließt, wobei der Teilabschnitt (610) bevorzugt eine Entlüftungslinie bildet, die mit ihrem anderen Ende mit der Atmosphäre verbunden ist.

9. Gerät nach einem der vorstehenden Ansprüche, **dadurch gekennzeichnet, dass** die Gasanalysiervorrichtung (6) ein Messelement (620) mit mehreren Sensorkanälen umfasst, um gleichzeitig die NO- und NO₂-Konzentration zu messen.

10. Gerät nach einem der vorstehenden Ansprüche, **dadurch gekennzeichnet**, das die Gasanalysiervorrichtung (6) ein oder mehrere Infrarot-Messelemente (620) umfasst.

11. Gerät nach einem der vorstehenden Ansprüche, **dadurch gekennzeichnet, dass** es sich bei dem vierten Magnetventil (645) um ein 3-Wege-Magnetventil handelt, von dem ein Weg (650) mit der Atmosphäre verbunden ist.

12. Gerät nach einem der vorstehenden Ansprüche, **dadurch gekennzeichnet, dass** die Entnahmelinie (660) des Weiteren eine Pumpe (640) und einen Durchflussfühler (635) umfasst, die an die Steuervorrichtung (70) angeschlossen sind.

13. Gerät nach einem der vorstehenden Ansprüche, **dadurch gekennzeichnet, dass** die Gaseinspritzlinie (4) des Weiteren einen Druckfühler (49) umfasst, der an die Steuervorrichtung (70) angeschlossen ist.

14. Gerät nach einem der vorstehenden Ansprüche, **dadurch gekennzeichnet, dass** die Gaseinspritzlinie (4), die Gasanalyselinie (660) und die Steuervorrichtung (70) und die Kalibrierlinie (605) innerhalb eines einzigen Gehäuses angeordnet sind.

15. Anlage zur Verteilung eines NO-haltigen Gases, umfassend:
- einen Lüfter (9), um ein sauerstoffhaltiges Atemgas zuzuführen,
- einen Patientenkreislauf (630), der den Lüfter (9) mit einem Patienten verbindet,
- ein Gerät nach einem der vorstehenden Ansprüche, das über die Gaseinspritzlinie (4) und die Entnahmelinie (660) strömungstechnisch an den Patientenkreislauf (630) angeschlossen ist, und
- eine Quelle von gasförmigem NO (30), die die Gaseinspritzlinie (4) des Gerätes mit einem NO-haltigen Gas speist.

## Claims

1. Apparatus for dispensing gas comprising:
- a gas injection line (4) comprising a gas inlet (4a) and a gas outlet (4b), and enabling a gas to be transported from the gas inlet (4a) to the gas outlet (4b),
- a gas analysis line (660) operatively connecting a gas analysis device (6) to a gas sample inlet (6a),
- a driving device (70) controlling the passing of gas in the gas injection line (4) and the sampling of gas through the gas analysis line (660),
**characterised in that**:
- it comprises, additionally, a calibration line (605) operatively connected (701; 700) to the gas injection line (4) and to the gas analysis line (660) in a way to enable a direct passing of gas from the gas injection line (4) to the gas analysis line (660),
- the gas injection line (4) comprises a first magnetic valve (45) controlled by the driving device (70),
- the calibration line (605) comprises a second magnetic valve (600) controlled by the driving device (70),
- the gas analysis line (660) comprises a fourth magnetic valve (645), located between the sample inlet (6a) and the second magnetic valve (600), and
- the gas analysis device (6) is connected to the gas analysis line (660), between said second (600) and fourth magnetic valves (645).

2. Apparatus according to the preceding claim, **characterised in that** it comprises, additionally, a backup line (5) operatively connected to the gas injection line (4), above and below the first magnetic valve (45) disposed on said gas injection line (4).

3. Apparatus according to the preceding claim, **characterised in that** said backup line (5) comprises a third magnetic valve (51) controlled by the driving device (70).

4. Apparatus according to one of the preceding claims, **characterised in that** it comprises, additionally, a multi-channel magnetic valve (47) disposed on the gas injection line (4) between the first magnetic valve (45) and the gas inlet (4a), and to which the calibration line (605) and/or the backup line (5) is connected.

5. Apparatus according to one of the preceding claims, **characterised in that** the multi-channel magnetic valve (47) is a 3-channel magnetic valve, preferably the multi-channel magnetic valve (47) is controlled by the driving device (70).

6. Apparatus according to one of the preceding claims, **characterised in that** the driving device (70) comprises an electronic microprocessor card.

7. Apparatus according to one of the preceding claims, **characterised in that** the gas analysis device (6) comprises several measuring elements (615, 620, 625) enabling the NO, NO₂ and O₂ concentration of said measuring elements (615, 620, 625) to be measured, being connected to the driving device (70), preferably the gas analysis device (6) comprises a first measuring element (615) enabling the NO concentration to be measured, a second measuring element (620) enabling the NO and NO₂ concentrations to be simultaneously measured, and a third measuring element (625) enabling the O₂ concentration to be measured.

8. Apparatus according to one of the preceding claims, **characterised in that** the gas analysis device (6) comprises several measuring elements (615, 620, 625) enabling the NO, NO₂ and O₂ concentration of said measuring elements (615, 620, 625) to be measured, being disposed on a section (610) coming to be operatively connected to the sampling line (660), preferably said section (610) constitutes a surrounding breather line connected by its other end to the air.

9. Apparatus according to one of the preceding claims, **characterised in that** the gas analysis device (6) comprises a measuring element (620) with several detection channels, in order to simultaneously measure NO and NO₂ concentration.

10. Apparatus according to one of the preceding claims, **characterised in that** the gas analysis device (6) comprises one or several infrared measuring elements (620).

11. Apparatus according to one of the preceding claims, **characterised in that** said fourth magnetic valve (645) is a 3-channel magnetic valve, of which one channel (650) is connected to the air.

12. Apparatus according to one of the preceding claims, **characterised in that** the sampling line (660) comprises additionally a pump (640) and a flow sensor (635) connected to the driving device (70).

13. Apparatus according to one of the preceding claims, **characterised in that** the gas injection line (4) comprises additionally a pressure sensor (49) connected to the driving device (70).

14. Apparatus according to one of the preceding claims, **characterised in that** the gas injection line (4), the gas analysis line (660) and the driving device (70) and the calibration line (605) are disposed within one single case.

15. Dispensing facility of a gas containing NO, comprising:
- a ventilator (9) to deliver a breathing gas containing oxygen,
- a patient circuit (630) connecting the ventilator (9) to a patient,
- an apparatus according to one of the preceding claims operatively connected to said patient circuit (630) via the gas injection line (4) and the sampling line (660), and
- a gaseous NO source (30) supplying the gas injection line (4) of the apparatus with a gas containing NO.
